(19) Europäisches Patentamt
European Patent Office
Office européen des brevets

(11) EP 4 249 040 A1

(12) EUROPÄISCHE PATENTANMELDUNG

(43) Veröffentlichungstag:
27.09.2023 Patentblatt 2023/39

(21) Anmeldenummer: 22163254.0

(22) Anmeldetag: 21.03.2022

(51) Internationale Patentklassifikation (IPC):
A61M 60/122 (2021.01)    A61M 60/216 (2021.01)
A61M 60/422 (2021.01)    A61M 60/538 (2021.01)
A61M 60/546 (2021.01)    A61M 60/822 (2021.01)

(52) Gemeinsame Patentklassifikation (CPC):
A61M 60/178; A61M 60/232; A61M 60/422;
A61M 60/538; A61M 60/546; A61M 60/822

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
KH MA MD TN

(71) Anmelder: Berlin Heart GmbH
12247 Berlin (DE)

(72) Erfinder:
• Peters, Oliver
14129 Berlin (DE)

• Kiesner, Matthias
15834 Rangsdorf (DE)
• Steingräber, Robert
14055 Berlin (DE)

(74) Vertreter: Pfenning, Meinig & Partner mbB
Patent- und Rechtsanwälte
Joachimsthaler Straße 10-12
10719 Berlin (DE)

Bemerkungen:
Die Patentansprüche 16 bis 19 gelten als fallen
gelassen, da die entsprechenden
Anspruchsgebühren nicht entrichtet wurden (R. 45(3)
EPÜ).

(54) **STEUEREINHEIT FÜR EINE BLUTPUMPE UND PUMPENSYSTEM**

(57) Die Anmeldung betrifft eine Steuereinheit (200) für eine Blutpumpe (300), ein Pumpensystem (100) mit einer solchen Steuereinheit (200) sowie ein Verfahren zum Ansteuern einer Blutpumpe (300). Die Blutpumpe (300) umfasst einen magnetisch gelagerten Rotor (320) und einen Stator (340) zum Ausüben einer Lagerkraft entlang einer Lagerwirkrichtung und eines Drehmoments auf den Rotor (320) und ist eingerichtet zum Bereitstellen eines Messsignals, umfassend einen von einer Bewegung des Rotors (320) entlang der Lagerwirkrichtung abhängigen Anteil und einen von einer Rotation des Rotors (320) abhängigen Anteil. Die Steuereinheit (200) ist eingerichtet, basierend auf dem Messsignal ein Steuersignal derart zu bestimmen, dass der Rotor (320) berührungsfrei in einem Gehäuse (301) gelagert und eine Rotation des Rotors (320) geregelt wird, wobei eine Vorgabe für eine Änderungsrate der Rotationsgeschwindigkeit der Rotation des Rotors (320) um die Rotationsachse (X) durch eine vorgegebene Maximaländerungsrate nach oben begrenzt ist, und den Stator (340) gemäß dem bestimmten Steuersignal anzusteuern.

Fig. 1

EP 4 249 040 A1

**Beschreibung**

Die Anmeldung betrifft eine Steuereinheit für eine Blutpumpe, insbesondere eine Rotationsfluidpumpe zur Herzunterstützung, ein Pumpensystem mit einer solchen Steuereinheit sowie ein Verfahren zum Ansteuern einer Blutpumpe.

Eine mittels einer anmeldungsgemäßen Steuereinheit ansteuerbare Blutpumpe umfasst einen in einem Gehäuse magnetisch gelagerten und zum Fördern eines Fluids um eine Rotationsachse rotierbaren Rotor und einen Stator, wobei der Stator eingerichtet ist zum Erzeugen eines variierbaren Statormagnetfelds zum Ausüben einer variierbaren Lagerkraft entlang einer Lagerwirkrichtung und eines Drehmoments bezüglich der Rotationsachse auf den Rotor, wobei die Blutpumpe eingerichtet ist zum Bereitstellen eines Messsignals, das von einer Position und/oder Bewegung des Rotors in dem Gehäuse abhängt.

Blutpumpen dieser Art sowie entsprechende Steuereinheiten und Ansteuerverfahren sind aus dem Stand der Technik bekannt.

Bei der Ansteuerung einer solchen Blutpumpe kann es vorgesehen sein, basierend auf dem Messsignal ein Steuersignal zum Variieren des Statormagnetfelds derart zu bestimmen, dass der Rotor mittels der Lagerkraft berührungsfrei entlang der Lagerwirkrichtung in dem Gehäuse gelagert und mittels des Drehmoments eine Rotation des Rotors um die Rotationsachse erzeugt wird.

Dabei können verschiedene Einflüsse auftreten, die die erforderlichen Schritte zum Bestimmen eines Steuersignals zum Ansteuern der berührungsfreien Lagerung und der Rotation des Rotors erschweren. Dazu gehören etwa eine inhärent komplexe Dynamik der Blutpumpe als anzusteuerndes System, Instabilitäten dieses Systems, systematische und stochastische Messfehler sowie mögliche Kopplungen verschiedener Messparameter und/oder Systemfreiheitsgrade. Lassen sich beispielsweise bei der Auswertung des genannten Messsignals rotationsabhängige und translationsabhängige Anteile des Messsignals nicht eindeutig voneinander trennen, so kann dies die Zuverlässigkeit einer Regelung zur Stabilisierung rotatorischer und translatorischer Freiheitsgrade beeinträchtigen.

Vor dem Hintergrund des Stands der Technik liegt der Anmeldung die Aufgabe zugrunde, eine Steuereinheit für eine Blutpumpe, ein Pumpensystem und ein Verfahren zum Ansteuern einer Blutpumpe bereitzustellen, die die genannten Probleme verringern oder vermeiden und insbesondere einen zuverlässigen und sicheren Betrieb der Blutpumpe im Hinblick auf eine Regelung der berührungsfreien Lagerung und der Rotation des Rotors ermöglichen.

Zur Lösung der Aufgabe werden eine Steuereinheit nach Anspruch 1, ein Pumpensystem nach Anspruch 14 und ein Verfahren nach Anspruch 18 vorgeschlagen. Vorteilhafte Ausgestaltungen und Weiterbildungen ergeben sich in Verbindung mit den Merkmalen der Unteransprüche.

Die vorgeschlagene Steuereinheit ist eingerichtet zum Ansteuern einer Blutpumpe der oben genannten Art, wobei das Messsignal einen von einer Lagerposition und/oder Lagergeschwindigkeit einer Bewegung des Rotors entlang der Lagerwirkrichtung abhängigen Anteil (im Folgenden auch translationsabhängiger Anteil des Messsignals) und einen von einem Rotationswinkel und/oder einer Rotationsgeschwindigkeit einer Rotation des Rotors um die Rotationsachse abhängigen Anteil (im Folgenden auch rotationsabhängiger Anteil des Messsignals) umfasst. Das Messsignal kann dabei ein ein- oder mehrdimensionales Messsignal sein, kann also beispielsweise ein einziges Sensorsignal oder mehrere gleichartige oder verschiedene Sensorsignale und/oder davon abgeleitete Signale umfassen.

Das Steuersignal ist typischerweise ein mehrdimensionales Signal. Wenn die Lagerkraft und das Drehmoment mittels im Wesentlichen unabhängiger Aktoren oder Aktorkomponenten erzeugt werden, so können ein rotatorischer Anteil des Steuersignals und ein translatorischer Anteil des Steuersignals verschiedenen vektoriellen Komponenten des Steuersignals entsprechen. Werden Lagerkraft und Drehmoment dagegen mittels gekoppelter Aktoren oder Aktorkomponenten erzeugt, die also jeweils sowohl die Lagerkraft als auch das Drehmoment beeinflussen, so können eine oder mehrere vektorielle Komponenten des Steuersignals jeweils sowohl einen translatorischen Anteil als auch einen rotatorischen Anteil umfassen.

Die Blutpumpe ist vorzugsweise eingerichtet zum Fördern des Fluids, insbesondere Blut, von einem Einlass zu einem Auslass. Der Einlass und der Auslass können jeweils direkt oder indirekt, insbesondere mittels einer Kanüle, mit einem Herzen und/oder einem Blutgefäß verbindbar sein. Die Blutpumpe kann insbesondere als VAD (ventricular assist device) einsetzbar sein.

Die Steuereinheit ist dazu eingerichtet, basierend auf dem Messsignal ein Steuersignal zum Variieren des Statormagnetfelds derart zu bestimmen, dass der Rotor mittels der Lagerkraft berührungsfrei entlang der Lagerwirkrichtung in dem Gehäuse gelagert und mittels des Drehmoments eine Rotation des Rotors um die Rotationsachse erzeugt, insbesondere gesteuert und/pder geregelt, wird, wobei eine Vorgabe für eine Änderungsrate der Rotationsgeschwindigkeit der Rotation des Rotors um die Rotationsachse durch eine vorgegebene Maximaländerungsrate nach oben begrenzt ist, um einen Einfluss einer Änderung der Rotationsgeschwindigkeit auf das bestimmte Steuersignal zu begrenzen.

Das Steuersignal kann dabei einen translatorischen Anteil zum berührungsfreien Lagern des Rotors entlang der Lagerwirkrichtung in dem Gehäuse und einen rotatorischen Anteil zum Regeln der Rotation des Rotors umfassen.

Dass die Vorgabe für die Änderungsrate der Rotationsgeschwindigkeit begrenzt wird, um den Einfluss der Änderung der Rotationsgeschwindigkeit auf das bestimmte Steuersignal zu begrenzen ist so zu verstehen, dass eine Abhängigkeit des Steuersignals von der Änderung der Rotationsgeschwindigkeit wenigstens zeitweise geringer ist als bei einem Bestimmen des Steuersignals ohne Begrenzen der Änderungsrate und/oder dass eine maximale Anhängigkeit des Steuersignals von der Änderung der Rotationsgeschwindigkeit geringer ist als bei einem Bestimmen des Steuersignals ohne Begrenzen der Änderungsrate.

Durch Begrenzen der Vorgabe für die Änderungsrate der Rotationsgeschwindigkeit in der genannten Weise kann es ermöglicht werden, den rotatorischen Anteil des Steuersignals und den translatorischen Anteil des Steuersignals dynamisch jedenfalls teilweise zu entkoppeln, so dass das Lagern des Rotors entlang der Lagerwirkrichtung vergleichsweise schnell, das Regeln der Rotation des Rotors vergleichsweise langsam erfolgt. So kann eine Zuverlässigkeit und Sicherheit der Blutpumpe im Hinblick auf die Regelung der berührungsfreien Lagerung und der Rotation des Rotors verbessert werden. Dies gilt insbesondere dann, wenn sich beispielsweise bei der Auswertung des genannten Messsignals rotationsabhängige und translationsabhängige Anteile des Messsignals nicht eindeutig voneinander trennen lassen, was etwa der Fall sein kann, wenn mehrere verschiedene Systemzustände der Blutpumpe zu identischen oder jedenfalls im Rahmen der Messgenauigkeit ununterscheidbaren oder nahezu unterscheidbaren Werten und/oder Zeitverläufen des Messsignals führen können. Ein solches System wird im Folgenden als mehrdeutiges System bezeichnet. Die Steuereinheit ist ferner dazu eingerichtet, den Stator gemäß dem bestimmten Steuersignal anzusteuern. Die Steuereinheit ist vorzugsweise dazu eingerichtet, ein Erfassen des Messsignals, das Bestimmen des Steuersignals und das entsprechende Ansteuern des Stators mehrfach wiederholt in einer Abfolge von Ansteuerzyklen durchzuführen.

Die vorgegebene Maximaländerungsrate kann insbesondere so gewählt sein, dass der Einfluss der Änderung der Rotationsgeschwindigkeit durch das Ansteuern des Stators gemäß dem bestimmten Steuersignal auf das Steuersignal, insbesondere auf eine basierend auf dem Messsignal vorgenommene und in das Steuersignal eingehende Bestimmung der Lagerposition und/oder Lagergeschwindigkeit, begrenzt wird gegenüber einem Ansteuern des Stators gemäß einem Steuersignal, das ohne Begrenzen der Änderungsrate der Rotationsgeschwindigkeit auf die vorgegebene Maximaländerungsrate bestimmbar ist.

Die Steuereinheit ist vorzugsweise dazu eingerichtet, das Steuersignal so zu bestimmen, dass eine vorgegebene und/oder tatsächliche und/oder gemessene Änderungsrate der Rotationsgeschwindigkeit des Rotors beim Ansteuern des Stators gemäß dem bestimmten Steuersignal die Vorgabe und/oder die vorgegebene Maximaländerungsrate nicht erreicht oder übersteigt.

Die vorgegebene Maximaländerungsrate kann insbesondere ein innerhalb der Steuereinheit gespeicherter oder speicherbarer Parameter, insbesondere ein steuerungs- oder Regelungsparameter, sein. Die vorgegebene Maximaländerungsrate kann eine werkseitig voreingestellte und/oder im Betrieb unveränderliche Vorgabe sein. Die Steuereinheit kann dazu eingerichtet sein, die vorgegebene Maximaländerungsrate basierend auf einem oder mehreren Betriebsparametern der Blutpumpe zu bestimmen. Die Steuereinheit kann dazu eingerichtet sein, die vorgegebene Maximaländerungsrate in bestimmten Intervallen, beispielsweise in jedem Ansteuerzyklus oder nach einer vorgegebenen Anzahl von Ansteuerzyklen oder mehrfach in einem Ansteuerzyklus, neu zu bestimmen. Die vorgegebene Maximaländerungsrate kann alternativ oder zusätzlich durch eine oder mehrere konstruktive Eigenschaften, beispielsweise mechanische, elektrische und/oder magnetische Eigenschaften, der Blutpumpe gegeben sein, etwa durch eine Massenträgheit des Rotors und/oder ein maximal erzielbares Motordrehmoment.

Die vorgegebene Maximaländerungsrate (beispielhafte Einheit $rad/s^2$) kann proportional zu einem Dynamik-Parameter der Bewegung des Rotors entlang der Lagerwirkrichtung sein. Der Dynamik-Parameter kann ein Parameter einer Steuerung oder Regelung der Bewegung des Rotors entlang der Lagerwirkrichtung und/oder eine basierend auf dem Messsignal bestimmte Größe sein. Beispielsweise kann der Dynamik-Parameter eine Lagerbeschleunigung und/oder Auslenkungsfrequenz und/oder Auslenkungsfrequenz-Änderungsrate der Bewegung des Rotors entlang der Lagerwirkrichtung und/oder eine Durchtrittsfrequenz und/oder Resonanzfrequenz und/oder inverse Zeitkonstante einer Regelung der Bewegung des Rotors entlang der Lagerwirkrichtung sein.

Eine Proportionalitätskonstante, die die vorgegebene Maximaländerungsrate und den Dynamik-Parameter verknüpft, kann dabei so gewählt werden, dass die Bewegung des Rotors entlang der Lagerwirkrichtung unter Berücksichtigung eines maximalen Auslenkungsbereichs des Rotors entlang der Lagerwirkrichtung auf eine "Rotationsperiode", also auf einen Vollkreiswinkel, normiert wird, so dass die vorgegebene Maximaländerungsrate und der vorgegebene Dynamikparameter in identischen Einheiten miteinander vergleichbar sind. Somit kann beispielsweise die Lagerbeschleunigung in den Einheiten der Änderungsrate der Rotationsgeschwindigkeit (z. B. $rad/s^2$) dargestellt werden.

Die vorgegebene Maximaländerungsrate kann durch eine Zeitkonstante, insbesondere eine Verzugszeit und/oder Sprungantwortzeit und/oder Anstiegszeit, einer Regelung der Rotation des Rotors gegeben sein. Beispielsweise kann die Rotationsgeschwindigkeit des Rotors mittels eines langsamen Reglers, etwa eines Reglers ohne D-Glied, geregelt werden. Dann kann sich die Rotationsgeschwindigkeit des Rotors nicht schneller ändern, als es eine Zeitkonstante, etwa die Sprungantwortzeit, des Reglers erlaubt, womit die vorgegebene Maximaländerungsrate durch diese Zeitkonstante gegeben und die Vorgabe für die Änderungsrate der Rotationsgeschwindigkeit der Rotation des Rotors entsprechend nach oben begrenzt ist.

Es kann vorgesehen sein, dass die vorgegebene Maximaländerungsrate höchstens die Hälfte, vorzugsweise höchstens ein Fünftel, insbesondere höchstens ein Zehntel einer charakteristischen Änderungsrate, etwa der Auslenkungsfrequenz-Änderungsrate oder der Lagerbeschleunigung, der Bewegung des Rotors entlang der Lagerwirkrichtung beträgt. Es kann vorgesehen sein, dass eine charakteristische Zeitkonstante, durch die die vorgegebene Maximaländerungsrate gegeben ist, mindestens das Doppelte, vorzugsweise das Fünffache, insbesondere mindestens das Zehnfache einer charakteristischen Zeitkonstante der Bewegung des Rotors entlang der Lagerwirkrichtung beträgt.

Zur Illustration möglicher Kenngrößen der Systemdynamik werden im Folgenden einige mögliche Werte der vorgegebenen Maximaländerungsrate und verschiedener Dynamik-Parameter angegeben. Die angegebenen Werte und Wertebereiche sind als Beispiele zu verstehen; abweichende Werte und Wertebereiche sind möglich. Eine Rotationsgeschwindigkeit des Rotors (im Folgenden auch Rotordrehzahl bzw. Drehzahl) kann beispielsweise im Bereich von 1000 bis 4000 rpm veränderlich sein. Eine Rotordrehzahl von 4000 rpm entspricht etwa 420 rad/s oder 66 Hz. Die Maximaländerungsrate kann beispielsweise im Bereich von 100 rpm/ms bis 1000 rpm/ms liegen. Eine bevorzugte Maximaländerungsrate von 400 rpm/ms entspricht etwa 42000 rad/s$^2$. Die Lagergeschwindigkeit kann insbesondere im Bereich vom 0,1°m/s bis 1 m/s, beispielsweise bei ca. 0.5 m/s liegen. Die Lagerbeschleunigung kann insbesondere im Bereich vom 50 m/s$^2$ bis 1000 m/s$^2$, beispielsweise bei ca. 500 m/s$^2$ liegen. Als Grenzfrequenz für die Ansteuerung der Rotations-Änderungsrate kann etwa ein Wert von weniger als 50 Hz, beispielsweise ca. 20 Hz vorgesehen sein. Als Grenzfrequenz für die Ansteuerung der Lagerbeschleunigung kann etwa ein Wert von mehr als 50 Hz, beispielsweise ca. 100 Hz vorgesehen sein.

Das Messsignal kann ein ein- oder mehrdimensionales Messsignal sein, kann also beispielsweise ein einziges Sensorsignal oder mehrere gleichartige oder verschiedene Sensorsignale und/oder davon abgeleitete Signale umfassen. Das Messsignal kann einer aufgrund einer durch die Rotation des Rotors und/oder die Bewegung des Rotors entlang der Lagerwirkrichtung verursachten magnetischen Feldänderung im Stator induzierten elektromotorischen Gegenkraft (Back-EMF) entsprechen. Das der Back-EMF entsprechende Messsignal kann ohne zusätzlichen Sensor mittels Motorspulenwicklungen des Stators erfassbar sein, was also eine "sensorlose" Erzeugung des Messsignals ermöglicht. Ein Ansteuern eines Systems mittels eines Reglers basierend auf einer sensorlosen Erzeugung eines Messsignals wird auch als sensorlose Regelung bezeichnet und insbesondere bei bürstenlosen Gleichstrommotoren (BLDC-Motoren) angewandt. Rotor und Stator der Blutpumpe können einen BLDC-Motor bilden.

Hier soll noch auf einen Unterschied der sensorlosen Regelung im Sinne dieser Anmeldung gegenüber sensorloser Regelung von BLDC-Motoren gemäß dem Stand der Technik eingegangen werden. Aus dem Stand der Technik der sensorlosen Regelung von BLDC-Motoren ist eine eine sensorlose Kommutierung bekannt. Hierbei wird der Rotationswinkel des Rotors sensorlos auf Basis der Back-EMF bestimmt, und ein elektrischer Kommutator oder eine feldorientierte Regelung generiert die notwendigen Phasensignale um den Motor zu rotieren. In konventionellen Motoren, welche z. Bsp. mit Kugellager gelagert sind, ist der durch die Rotormagnete generierte magnetische Fluss im Stator in erster Näherung konstant oder ändert sich nur langsam mit der Temperatur der Rotormagnete. In einem Axialflussmotor ist der magnetische Fluss zusätzlich abhängig von der axialen Rotorposition (Lagerposition). Ist hierbei die axiale Rotorposition variabel, dann lässt sich aus dem magnetischen Fluss auf die axiale Position schließen. Bei einer auf Back-EMF basierenden "sensorlosen" Erfassung im Sinne dieser Anmeldung wird die Änderung des vom Rotor erzeugten magnetischen Flusses, welcher die Statorspulen durchfließt, über der Zeit ermittelt (die Statorspulen werden also als Sensor verwendet). Hierbei kann eine Rotationsschwingung ein ähnliches Induktionssignal wie eine axiale Translationsschwingung generieren, wodurch das System also mehrdeutig sein kann.

Bei der Verwendung eines sensorlos bereitgestellten Messsignals kann eine Trennung der rotationsabhängigen und translationsabhängigen Anteile des Messsignals folglich besonders erschwert sein. Das dynamische Entkoppeln der rotatorischen und translatorischen Anteile des Steuersignals im oben beschriebenen Sinne kann daher den Betrieb einer sensorlosen Blutpumpe verbessern und somit auch zu einem besonders kompakten und robusten Design der Blutpumpe beitragen. Bei der sensorlosen Erzeugung des Messsignals ist ferner die Anzahl von Adern, die in einer Driveline zum Übertragen des Messsignals vorgesehen sein müssen, besonders gering, was ebenfalls ein robustes und kompaktes Design und/oder ein vereinfachtes Vorsehen von Redundanz ermöglichen kann.

Es kann vorgesehen sein, dass eine Mehrzahl von Messpunkten, entsprechend dem Messsignal zu einer Mehrzahl von Zeitpunkten, erfasst wird und die Steuereinheit dazu eingerichtet ist, das Steuersignal basierend auf der Mehrzahl von Messpunkten zu bestimmen. Auf diese Weise wird also ein Zeitverlauf des Messsignals über die Mehrzahl von Zeitpunkten bei der Bestimmung des Steuersignals berücksichtigt, was insbesondere bei einem mehrdeutigen System dazu beitragen kann, die Mehrdeutigkeit zu verringern und somit ein zuverlässigeres Ansteuern der Blutpumpe zu ermöglichen. Die Messung eines Messpunkts zu einem entsprechenden Zeitpunkt wird auch als Zeitschritt oder Abtastschritt, das zwischen zwei Messpunkten liegende Zeitintervall als Abtastzeit bezeichnet.

Die Steuereinheit kann dazu eingerichtet sein,

basierend auf dem Messsignal einen oder mehrere Zustandsparameter eines Modells der Bewegung des Rotors, insbesondere der Rotation des Rotors um die Rotationsachse und/oder der Bewegung des Rotors entlang der Lagerwirkrichtung, zu schätzen, wobei der bzw. die Zustandsparameter insbesondere den Rotationswinkel und/oder die Rotationsgeschwindigkeit und/oder die Änderungsrate der Rotationsgeschwindigkeit und/oder eine Rotationsfrequenz und/oder eine Rotationsfrequenz-Änderungsrate der Rotation des Rotors und/oder die Lagerposition und/oder die Lagergeschwindigkeit und/oder eine Lagerbeschleunigung und/oder eine Auslenkungsfrequenz und/oder eine Auslenkungsfrequenz-Änderungsrate der Bewegung des Rotors entlang der Lagerwirkrichtung und/oder die Lagerkraft und/oder das Drehmoment und/oder eine Feldstärke eines innerhalb des Stators durch den Rotor erzeugten Magnetfeldes und/oder eine zeitliche Änderung dieser Feldstärke umfassen und

das Steuersignal basierend auf einem oder mehreren des bzw. der geschätzten Zustandsparameter zu bestimmen. Dass Schätzen des bzw. der Zustandsparameter des Modells wird mittels eines Beobachters bzw. durch Beobachtung (im Sinne der Regeltechnik) vorgenommen und kann auf verschiedene Arten erfolgen.

Die Steuereinheit kann etwa dazu eingerichtet sein, den bzw. die Zustandsparameter unter Verwendung eines Extended Kalman Filters (EKF) zu schätzen. Ein EKF eignet sich besonders für die Beobachtung eines nichtlinearen dynamischen Systems.

Es kann vorgesehen sein, dass der bzw. die Zustandsparameter einen Drehmomentverstärkungsfaktor umfassen, der einem Verhältnis der Rotationsgeschwindigkeit und des Drehmoments entspricht. Berücksichtigung dieses Zustandsparameters kann für die Auslegung des Beobachters vorteilhaft sein, wie weiter unten anhand von Beispielen illustriert wird.

Die Steuereinheit kann dazu eingerichtet sein, den bzw. die Zustandsparameter unter Verwendung eines manuell ausgelegten Beobachters zu schätzen. Durch manuelle Auslegung kann ein für die Beobachtung erforderlicher Rechenaufwand gegenüber einem Schätzverfahren wie etwa dem EKF verringert werden.

Die Steuereinheit kann dazu eingerichtet sein, den bzw. die Zustandsparameter unter Verwendung einer vektoriellen Zerlegung einer gemessenen Back-EMF in eine Rotationskomponente und eine Lagerpositionskomponente zu schätzen, was eine besonders einfache Schätzung ermöglicht, wie weiter unten näher erläutert wird.

Die Steuereinheit kann dazu eingerichtet sein, dass Steuersignal so zu bestimmen, dass der Rotor in eine Ziel-Lagerposition eingeregelt wird, an der auf den Rotor wirkende äußere Kräfte entlang der Lagerwirkrichtung sich zu einer vorgegebenen Kraft, insbesondere einer Nullkraft, addieren und/oder an der eine zum Erzeugen des variierbaren Statormagnetfelds aufgewandte Leistung minimal ist.

Die Steuereinheit kann ferner dazu eingerichtet sein,

eine Mehrzahl von Messpunkten, entsprechend wenigstens eines Anteils des Messsignals zu einer Mehrzahl von Zeitpunkten bei bekannter, insbesondere konstanter, Rotationsgeschwindigkeit und konstanter Lagerposition des Rotors zu erfassen,

eine periodische Korrekturfunktion, insbesondere eine Sinusfunktion oder mehrere überlagerte Sinusfunktionen, an wenigstens einen Anteil des Messsignals mittels Optimierung anzupassen.

Auf Grundlage der so angepassten Korrekturfunktion kann eine von dem Rotationswinkel des Rotors abhängige, insbesondere periodisch mit einer Periode der Rotation des Rotors auftretende, Störung des Messsignals ausgeglichen und somit deren Einfluss auf das Steuersignal verringert werden. Eine solche Störung kann etwa aufgrund von Asymmetrien des Systems, beispielsweise des Rotormagnetfelds und/oder der Motorspulen, auftreten.

Das vorgeschlagene Pumpensystem umfasst eine Blutpumpe und eine Steuereinheit der vorgeschlagenen Art, eingerichtet zum Ansteuern der Blutpumpe,

wobei die Blutpumpe einen in einem Gehäuse magnetisch gelagerten und zum Fördern eines Fluids um eine Rotationsachse rotierbaren Rotor und einen Stator umfasst, wobei der Stator eingerichtet ist zum Erzeugen eines variierbaren Statormagnetfelds zum Ausüben einer variierbaren Lagerkraft entlang einer Lagerwirkrichtung und eines Drehmoments bezüglich der Rotationsachse auf den Rotor;

wobei die Blutpumpe eingerichtet ist zum Bereitstellen eines Messsignals, umfassend einen von einer Lagerposition und/oder Geschwindigkeit einer Bewegung des Rotors entlang der Lagerwirkrichtung abhängigen Anteil und einen von einem Rotationswinkel und/oder einer Rotationsgeschwindigkeit einer Rotation des Rotors um die Rotationsachse abhängigen Anteil.

In dem vorgeschlagenen Pumpensystem entfaltet die vorgeschlagene Steuereinheit ihre oben beschriebenen Vorteile.

Die Steuereinrichtung eignet sich für verschiedene Arten von Blutpumpen, die sich etwa in der Art des aus Rotor und Stator gebildeten Elektromotors und/oder Lagerwirkrichtung unterscheiden können. Der Rotor und der Stator können beispielsweise einen Axialflussmotor oder Radialflussmotor bilden, wobei die Lagerwirkrichtung in einem von Null verschiedenen Winkel, insbesondere im Wesentlichen senkrecht, zu der Rotationsachse (X) des Rotors (320) stehenden Radialrichtung steht. Der Rotor (320) kann neben der vorgenannten Lagerwirkrichtung auch entlang einer zweiten Lagerwirkrichtung aktiv magnetisch in dem Gehäuse (301) gelagert sein.

Der Rotor kann ein entlang der Lagerwirkrichtung und/oder der Rotationsachse nichtlineares, insbesondere wenigstens näherungsweise exponentiell abklingendes, Permanentmagnetfeld erzeugen.

Das vorgeschlagene Verfahren ist vorgesehen zum Ansteuern einer Blutpumpe,

wobei die Blutpumpe einen in einem Gehäuse magnetisch gelagerten und zum Fördern eines Fluids um eine Rotationsachse rotierbaren Rotor und einen Stator umfasst, wobei der Stator eingerichtet ist zum Erzeugen eines variierbaren Statormagnetfelds zum Ausüben einer variierbaren Lagerkraft entlang einer Lagerwirkrichtung;
wobei die Blutpumpe eingerichtet ist zum Bereitstellen eines Messsignals, umfassend eine von einer Lagerposition und/oder Geschwindigkeit einer Bewegung des Rotors entlang der Lagerwirkrichtung abhängigen Anteil und einen von einem Rotationswinkel und/oder einer Rotationsgeschwindigkeit einer Rotation des Rotors um die Rotationsachse abhängigen Anteil.

Das Verfahren umfasst:

Bestimmen, basierend auf dem Messsignal, eines Steuersignals zum Variieren des Statormagnetfelds derart, dass der Rotor mittels der Lagerkraft berührungsfrei entlang der Lagerwirkrichtung in dem Gehäuse gelagert und mittels des Drehmoments eine Rotation des Rotors um die Rotationsachse erzeugt, insbesondere gesteuert und/oder geregelt, wird, wobei eine Vorgabe für eine Änderungsrate der Rotationsgeschwindigkeit der Rotation des Rotors um die Rotationsachse durch eine vorgegebene Maximaländerungsrate nach oben begrenzt ist, um einen Einfluss einer Änderung der Rotationsgeschwindigkeit auf das bestimmte Steuersignal zu begrenzen, und
Ansteuern des Stators gemäß dem bestimmten Steuersignal.

Das vorgeschlagene Verfahren kann insbesondere unter Verwendung einer Steuereinheit der oben vorgeschlagenen Art und/oder eines Pumpensystems der oben vorgeschlagenen ausgeführt und entsprechend den optionalen Merkmalen der Steuereinheit und des Pumpensystems weiter ausgestaltet werden.

Ausführungsbeispiele des Anmeldungsgegenstandes werden nachfolgend mit Bezugnahme auf Zeichnungen erläutert. Dabei zeigen:

Fig. 1 eine schematische Darstellung eines Pumpensystems mit einer Blutpumpe und einer Steuereinheit,

Fig. 2 Zeitverläufe eines simulierten Messsignals in verschiedenen Betriebszuständen eines Pumpensystems,

Fig. 3 eine schematische Darstellung einer Pumpenregelung,

Fig. 4a und Fig. 4b schematische Illustrationen einer vektoriellen Zerlegung einer Back-EMF,

Fig. 5 eine schematische Darstellung einer Pumpenregelung gemäß einem weiteren Beispiel.

Wiederkehrende und ähnliche Merkmale verschiedener Ausführungsformen sind in den Abbildungen mit identischen alphanumerischen Bezugszeichen versehen.

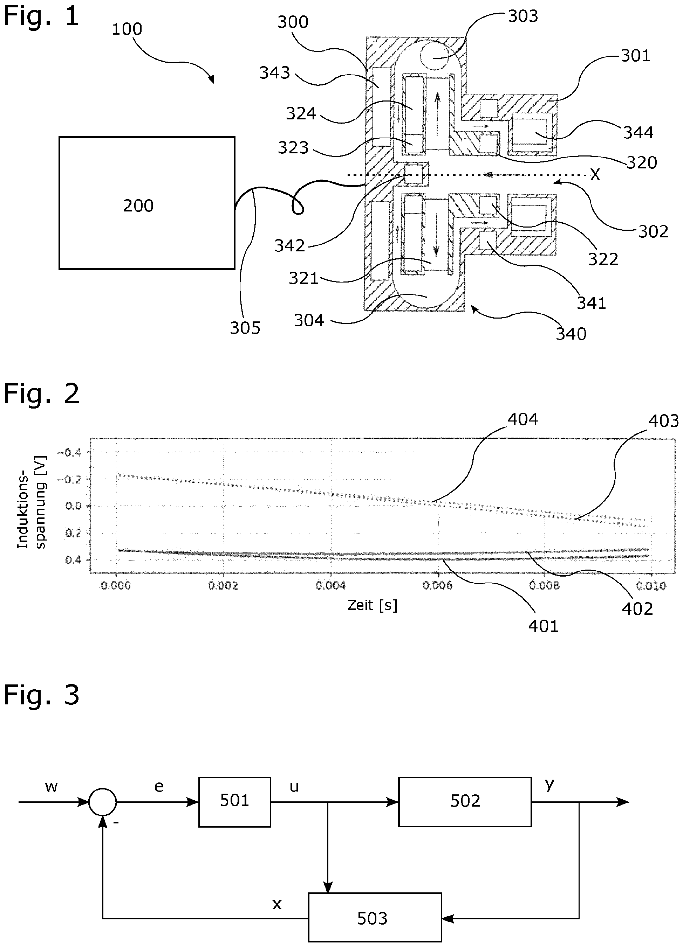

Das in Fig. 1 gezeigte Pumpensystem 100 umfasst eine Blutpumpe 300 und eine Steuereinheit 200, wobei die Steuereinheit 200 zum Ansteuern der Blutpumpe 300 eingerichtet ist.

Die Blutpumpe 300 ist als Rotationsfluidpumpe zur Herzunterstützung ausgebildet und umfasst einen in einem Gehäuse 301 magnetisch gelagerten und zum Fördern eines Fluids, insbesondere Blut, von einem Einlass 302 zu einem Auslass 303 um eine Rotationsachse X rotierbaren Rotor 320 und einen in dem Gehäuse 301 gebildeten Stator 340.

Der Einlass 302 ist im gezeigten Beispiel direkt (durch Implantation in eine Herzwand) mit einem Ventrikel eines Herzens verbindbar, der Auslass 303 ist mittels einer Kanüle, mit einem mit dem Herzen verbundenen Blutgefäß verbindbar. Die Blutpumpe ist somit als VAD einsetzbar.

Der Rotor 320 und der Stator 340 der Blutpumpe 340 bilden einen dreiphasigen BLDC-Motor. Der Stator 340 umfasst eine Motorspulenanordnung 343, der Rotor 320 eine Motormagnetanordnung 324, so dass durch Bestromen der Motorspulen ein variierbares Statormagnetfeld zum Ausüben einer variierbaren Lagerkraft entlang einer Lagerwirkrichtung und eines Drehmoments bezüglich der Rotationsachse X auf den Rotor 320 erzeugbar ist.

Die Lagerwirkrichtung ist im Wesentlichen parallel zur Rotationsachse X angeordnet und wird im Folgenden auch als Axialrichtung bezeichnet. Dazu senkrechte Richtungen werden demensprechend als Radial- bzw. Lateralrichtung bezeichnet. Die Lagerwirkrichtung kann in einer anderen Richtung als der Axialrichtung, etwa in Radial- oder Lateralrichtung oder einer zur Rotationsachse X in einem anderen Winkel stehenden Richtung, angeordnet sein.

Entlang der Lagerwirkrichtung, also der Axialrichtung, ist der Rotor 320 in dem Gehäuse 301 mittels der variierbaren Lagerkraft aktiv magnetisch gelagert. Die Motorspulenanordnung 343 und die Motormagnetanordnung 324 wirken hierbei als Lagermagnete. Zusätzlich oder alternativ kann der Stator 340 mindestens eine optionale Steuerspule 344 umfassen, die zum Ausüben der Lagerkraft auf mindestens einen Magneten des Rotors 320, im gezeigten Beispiel auf das einlassseitige Rotormagnetlager 323, wirkt. Das Lagersteuersignal entspricht einer der Motorspulenanordnung 343 und/oder der Steuerspule 344 zu beaufschlagenden Spannung.

Das Fluid tritt im Betrieb im Wesentlichen in Axialrichtung in das Gehäuse 301 und den Rotor 320 ein und wird durch eine Beschaufelung 321 in radialer Richtung in eine Volute 304 des Gehäuses 301 gefördert, von wo aus es den Auslass 303 erreicht. Der Blutfluss ist in Fig. 1 schematisch durch Pfeile verdeutlicht.

Die Blutpumpe 300 des gezeigten Beispiels ist also als Radial- bzw. Zentrifugalpumpe ausgeführt. Der Gegenstand der Anmeldung ist jedoch nicht auf diesen Pumpentyp beschränkt, sondern für verschiedene Arten von Rotationsfluidpumpen anwendbar. Beispielsweise kann die Blutpumpe eine Axialpumpe sein. Auch hinsichtlich in der Art des aus Rotor 320 und Stator 340 gebildeten Elektromotors ist die Steuereinheit 320 für verschiedene Pumpen geeignet. Im gezeigten Beispiel bilden der Rotor 320 und der Stator 340 einen Axialflussmotor, wobei die Lagerwirkrichtung im Wesentlichen der Axialrichtung entspricht. Der Rotor und der Stator können alternativ einen Radialflussmotor bilden, wobei die Lagerwirkrichtung im Wesentlichen der Radialrichtung entspricht.

Der Rotor 320 ist in dem Gehäuse 301 in allen Raumrichtungen magnetisch gelagert. In radialer Richtung ist der Rotor 320 passiv magnetisch gelagert. Dazu weist der Rotor 320 ein einlassseitiges Rotormagnetlager 322 und ein auslassseitiges Rotormagnetlager 323, der Stator 340 ein einlassseitiges Statormagnetlager 341 und ein auslassseitiges Statormagnetlager 342 auf, wobei das einlassseitige Statormagnetlager 341 auf das einlassseitige Rotormagnetlager 322, das auslassseitige Statormagnetlager 342 auf das auslassseitige Rotormagnetlager 323 wirkt. Wie oben beschrieben, ist der Rotor 321 in axialer Richtung aktiv magnetisch gelagert. Der Gegenstand der Anmeldung ist jedoch nicht auf die beschriebene Lagergeometrie beschränkt, sondern auf verschiedene Pumpen mit aktiver Magnetlagerung entlang wenigstens einer Lagerwirkrichtung anwendbar.

Die Blutpumpe 300 ist eingerichtet ist zum Bereitstellen eines Messsignals, das einen von einer Lagerposition und/oder Lagergeschwindigkeit einer Bewegung des Rotors 320 entlang der Lagerwirkrichtung abhängigen (translationsabhängigen) Anteil und einen von einem Rotationswinkel und/oder einer Rotationsgeschwindigkeit einer Rotation des Rotors 320 um die Rotationsachse X abhängigen (rotationsabhängigen) Anteil umfasst. Das Messsignal entspricht einer aufgrund einer durch die Rotation des Rotors 320 und/oder die Bewegung des Rotors 320 entlang der Lagerwirkrichtung verursachten magnetischen Feldänderung im Stator 340 induzierten elektromotorischen Gegenkraft (Back-EMF), wird also sensorlos erzeugt. Das Messsignal ist ein mehrdimensionales (vektorielles) Messsignal, dessen Komponenten den Induktionsspannungen in den drei Phasen der Motorspulenanordnung 343 entsprechen.

Die Blutpumpe 300 kann alternativ oder zusätzlich einen Sensor, beispielsweise einen Wirbelstromsensor und/oder einen Magnetfeldsensor, insbesondere einen Hall-Sensor, zum Bereitstellen des Messsignals umfassen.

Die Back-EMF kann mittels des Induktionsgesetzes wie folgt dargestellt werden:

$$V_{BEMF} = -\frac{d\varphi}{dt}N = -\frac{dB}{dt}AN \qquad \text{(Gleichung 1).}$$

Dabei ist $V_{BEMF}$ die Back-EMF die durch Veränderung des Magnetfelds durch die Bewegung des Rotors 320 in die Motorspulenanordnung 343 induzierte Spannung, $\varphi$ ein magnetischer Fluss, t die Zeit, N eine Windungszahl einer Spule der Motorspulenanordnung 343, B eine magnetische Flussdichte und A eine Fläche der Spule.

Die durch die Bewegung der Rotormagnete 322, 323, 324 in eine Motorspule der Motorspulenanordnung 343 induzierte Induktionsspannung $V_{BEMF}$ ist an den Anschlüssen der Spule überlagert durch einen ohmschen Anteil $V_R$, eine Selbstinduktionsspannung $V_{Lii}$ der Spule sowie eines Gegeninduktionsspannung $V_{Lij}$ für jede weitere Motorspule. An den Anschlüssen einer einzelnen Motorspule ist die Summe $V_{Coil,i}$ als Phasenspannung messbar.

$$V_{Coil} = V_{BEMF} + V_R + V_{Lii} + \sum_{j=0}^{n} V_{Lij} \qquad \text{(Gleichung 2).}$$

Aus den Phasenströmen $I_i$ lassen sich die überlagerten Komponenten bestimmen:

$$V_R = I_i R_i \qquad \text{(Gleichung 3a),}$$

$$V_{Lii} = L_i \frac{dI_i}{dt} \qquad \text{(Gleichung 3b),}$$

$$V_{Lij} = L_{ij} \frac{dI_j}{dt} \qquad \text{(Gleichung 3c).}$$

Sind die Phasenströme und Phasenspannungen bekannt, dann kann die Induktionsspannung $V_{BEMF}$ mit Gleichung 2 berechnet werden. An einem dreiphasigen Motor in Dreiecksschaltung oder Sternschaltung ohne Sternpunktanschluss kann nur die Summe mehrerer Phasenspannungen oder Phasenströme gemessen werden. In diesem Fall können anstatt der einzelnen drei Phasenspannungen die drei Summen von jeweils zwei Phasenspannungen ermittelt werden. Die dargelegten Verfahren werden hiervon nicht eingeschränkt.

Aus dem Stand der Technik sind Verfahren bekannt, welche mit einer verringerten Anzahl an Sensoren die Induktionsspannung ermitteln können. Hierbei wird beispielsweise die Anschlussspannung an den Motorleitungen mit dem Tastverhältnis einer Pulsweitenmodulation und der Versorgungsspannung abgeschätzt. Es sind dann nur noch zwei Strommessungen erforderlich um die Induktionsspannungen zu bestimmen. Für den Sonderfall $I_i=0$ kann die Induktionsspannung direkt gemessen werden.

Da von den drei Induktionsspannungen $\{V_A, V_B, V_C\}$ in einem mit drei Leitungen angeschlossenen Dreiphasen-Motor von außen betrachtet nur zwei unabhängig sind, können diese mittels Clarke-Transformation eindeutig auf ein zweiphasiges System $\{V_a, V_b\}$ wie folgt umgewandelt werden:

$$\begin{bmatrix} V_a \\ V_b \end{bmatrix} = \frac{2}{3} \begin{bmatrix} 1 & -\frac{1}{2} & -\frac{1}{2} \\ 0 & \frac{\sqrt{3}}{2} & \frac{\sqrt{3}}{2} \end{bmatrix} \begin{bmatrix} V_A \\ V_B \\ V_C \end{bmatrix} \qquad \text{(Gleichung 4).}$$

Fig. 2 zeigt simulierte Messsignalkurven 401, 402, 403, 404 für einen Betrieb der Blutpumpe 300, wobei die Messsignalkurven 401 und 402 der Induktionsspannung $V_a$, die Messsignalkurven 403 und 404 der Induktionsspannung $V_b$ entsprechen. Die Messsignalkurven 401 und 403 entsprechen einem ersten Betriebszustand, in dem der Rotor mit einer ersten Drehzahl um die Rotationsachse X rotiert und sich in einer ersten Lagerposition entlang der Lagerwirkrichtung befindet. Die Messsignalkurven 402 und 404 entsprechen einem zweiten Betriebszustand, in dem der Rotor mit einer zweiten, gegenüber der ersten Drehzahl verringerten Drehzahl um die Rotationsachse X rotiert und sich in einer zweiten, gegenüber der ersten Lagerposition verschobenen zweiten Lagerposition befindet, wobei zusätzlich eine axiale Rotorbewegung vorliegt. Die Blutpumpe wird hierbei mit einem Ansteuerzyklus von 50 µs Dauer betrieben, die Zeitachse von 0 bis 0,01 s entspricht also 200 Ansteuerzyklen.

Wie in Fig. 2 zu erkennen ist, kann dieselbe Konstellation von Induktionsspannungen (zum Zeitpunkt t = 0) durch verschiedene Kombinationen von Lagerposition und Drehzahl erreicht werden. Deshalb lassen sich bei der Auswertung des genannten Messsignals rotationsabhängige und translationsabhängige Anteile des Messsignals nicht eindeutig voneinander trennen, das System ist also mehrdeutig. Zwar driften die Werte im Laufe der Zeit auseinander, jedoch kann bei der Ansteuerung nicht die hier nötige Zeitverzögerung von ca. 5 ms (25 Ansteuerzyklen) abgewartet werden, bevor entscheidbar ist, ob das Drehmoment oder die Lagerkraft angepasst werden muss. In dieser Zeit könnte der Rotor 320 bereits mit einer Wand des Gehäuses 301 kollidieren, was im Betrieb der Blutpumpe 300 unbedingt vermieden werden muss. Die Entscheidung hinsichtlich der Anpassung von Drehmoment oder Lagerkraft wird daher durch dynamische Entkopplung der rotatorischen und translatorischen Anteile des Steuersignals wie folgt getroffen.

Die Steuereinheit 200 ist dazu eingerichtet, basierend auf dem Messsignal ein Steuersignal zum Variieren des Statormagnetfelds derart zu bestimmen, dass der Rotor 320 mittels der Lagerkraft berührungsfrei entlang der Lagerwirkrichtung in dem Gehäuse 301 gelagert und mittels des Drehmoments eine Rotation des Rotors 320 um die Rotationsachse X geregelt wird, wobei eine Vorgabe für eine Änderungsrate der Rotationsgeschwindigkeit der Rotation des Rotors 320 (Rotationsgeschwindigkeits-Änderungsrate) um die Rotationsachse durch eine vorgegebene Maximaländerungsrate nach oben begrenzt, um einen Einfluss einer Änderung der Rotationsgeschwindigkeit auf das bestimmte Steuersignal zu begrenzen. Die Rotation des Rotors 320 muss nicht in allen Fällen geregelt werden, es kann auch ein Steuern der Rotation vorgesehen sein.

Das Steuersignal ist dabei ein mehrdimensionales Signal, entsprechend den Phasenspannungen zum Ansteuern des BLDC-Motors. Das Steuersignal umfasst dabei einen rotarischen Anteil zum berührungsfreien Lagern des Rotors 320 entlang der Lagerwirkrichtung in dem Gehäuse 301 und einen translatorischen Anteil zum Regeln der Rotation des Rotors 320, wobei jede vektorielle Komponente des Steuersignals beide Anteile in gekoppelter Form enthält.

Wie oben erläutert, werden durch das Begrenzen der Rotationsgeschwindigkeits-Änderungsrate der rotatorische Anteil des Steuersignals und der translatorischen Anteil des Steuersignals dynamisch teilweise entkoppelt, so dass das Lagern des Rotors 320 entlang der Lagerwirkrichtung vergleichsweise schnell, das Regeln der Rotation des Rotors vergleichsweise langsam erfolgt und eine Zuverlässigkeit und Sicherheit der Blutpumpe 300 im Hinblick auf die Regelung der berührungsfreien Lagerung und der Rotation des Rotors 320 verbessert werden können.

Die Steuereinheit 200 ist ferner dazu eingerichtet, den Stator gemäß dem bestimmten Steuersignal anzusteuern. Die Steuereinheit 200 ist dazu eingerichtet, ein Erfassen des Messsignals, das Bestimmen des Steuersignals und das entsprechende Ansteuern des Stators 340 mehrfach wiederholt in einer Abfolge von Ansteuerzyklen durchzuführen. Es ist ferner vorgesehen, dass in jedem Ansteuerzyklus eine Mehrzahl von Messpunkten, entsprechend dem zu einer Mehrzahl von Zeitpunkten erfassten Messsignal, berücksichtigt wird, indem das Steuersignal basierend auf der Mehrzahl von Messpunkten bestimmt wird.

Die Steuereinheit 200 ist mit der Blutpumpe 300 mittels einer Driveline 305 zum Übertragen des Messsignals und des Steuersignals verbunden.

Die Steuereinheit 200 ist vorzugsweise dazu eingerichtet, das Steuersignal so zu bestimmen, dass der Rotor in eine Ziel-Lagerposition eingeregelt wird, an der auf den Rotor 320 wirkende äußere Kräfte entlang der Lagerwirkrichtung sich zu einer vorgegebenen Kraft, insbesondere einer Nullkraft, addieren und/oder an der eine zum Erzeugen des variierbaren Statormagnetfelds aufgewandte Leistung minimal ist (auch als Nullkraftregelung bezeichnet).

Wie den vorangehenden Ausführungen zu entnehmen ist, eignet sich die Steuereinheit 200 gemäß der vorstehend beschriebenen Beispiele insbesondere zum Ausführen eines Verfahrens zum Ansteuern der Blutpumpe 300, wobei das Verfahren umfasst:

Bestimmen, basierend auf dem Messsignal, eines Steuersignals zum Variieren des Statormagnetfelds derart, dass der Rotor 320 mittels der Lagerkraft berührungsfrei entlang der Lagerwirkrichtung in dem Gehäuse 301 gelagert und mittels des Drehmoments eine Rotation des Rotors 320 um die Rotationsachse X erzeugt, insbesondere gesteuert und/oder geregelt, wird, wobei eine Vorgabe für eine Änderungsrate der Rotationsgeschwindigkeit der Rotation des Rotors 320 um die Rotationsachse X durch eine vorgegebene Maximaländerungsrate nach oben begrenzt ist, um einen Einfluss einer Änderung der Rotationsgeschwindigkeit auf das bestimmte Steuersignal zu begrenzen, und

Ansteuern des Stators 340 gemäß dem bestimmten Steuersignal.

Die vorgegebene Maximaländerungsrate ist dabei so gewählt, dass der Einfluss der Änderung der Rotationsgeschwindigkeit durch das Ansteuern des Stators 340 gemäß dem bestimmten Steuersignal auf das Steuersignal, insbesondere auf eine basierend auf dem Messsignal vorgenommene und in das Steuersignal eingehende Bestimmung der Lagerposition und/oder Lagergeschwindigkeit, begrenzt wird gegenüber einem Ansteuern des Stators 340 gemäß einem Steuersignal, das ohne Begrenzen der Änderungsrate der Rotationsgeschwindigkeit auf die vorgegebene Maximaländerungsrate bestimmbar ist. Die Steuereinheit 200 ist vorzugsweise dazu eingerichtet, das Steuersignal so zu bestimmen, dass eine tatsächliche und/oder gemessene Änderungsrate der Rotationsgeschwindigkeit des Rotors beim Ansteuern des Stators gemäß dem bestimmten Steuersignal die Vorgabe und/oder die vorgegebene Maximaländerungsrate nicht erreicht oder übersteigt.

Die Steuereinheit 200 ist dazu eingerichtet, basierend auf dem Messsignal einen Zustandsvektor, umfassend mehrere Zustandsparameter eines Modells der Rotation des Rotors 320, zu schätzen und das Steuersignal basierend auf dem Zustandsvektor zu bestimmen. Dass Schätzen des bzw. der Zustandsparameter des Modells wird mittels eines Beobachters bzw. durch Beobachtung (im Sinne der Regeltechnik) vorgenommen. Der Beobachter kann hierbei verschiedene Ausgestaltungen annehmen.

Im Folgenden werden beispielhafte Pumpenregelungen anhand von Fig. 3 bis Fig. 5 erläutert.

Fig. 3 zeigt in einem ersten einen Beispiel einen grundlegenden Regelkreis der Pumpenregelung mit Beobachtung, umfassend einen Regler 501, eine Regelstrecke 502 (umfassend den Stator 340 und den Rotor 320) und einen Beobachter 503. Basierend auf einer Regelabweichung e bestimmt der Regler 501 einen Stellgrößenvektor u, der sowohl der Regelstrecke 502 als auch dem Beobachter 503 zugeführt wird. Die physikalische Antwort der Regelstrecke 502 bestimmt den Messgrößenvektor y, der ebenfalls dem Beobachter 503 zugeführt wird. Der Beobachter schätzt die Zustandsparameter des Zustandsvektors x, auf dessen Grundlage die Regelabweichung e als Abweichung von dem Führungsgrößenvektor w bestimmt wird.

In einem zweiten Beispiel der Pumpenregelung, dessen grundlegende Struktur ebenfalls wie in Fig 3 dargestellt ist, ist der Beobachter 503 dazu eingerichtet, die Zustandsparameter des Zustandsvektors unter Verwendung eines Extended Kalman Filters (EKF) zu schätzen.

Einem solchen EKF-basierten Beobachter liegen die folgenden Gleichungen zugrunde:

$$\frac{dx}{dt} = f(x) + g(x)\,u \qquad \text{(Gleichung 5a),}$$

$$y = h(x) \qquad \text{(Gleichung 5b).}$$

Dabei sind f(x) eine Übergangsfunktion welche die Zustandsänderung $\frac{dx}{dt}$ in Abhängigkeit des inneren Zustandes x und g(x) eine Steuerfunktion welche die Zustandsänderung in Abhängigkeit eines Stellgrößenvektor u beschreibt, h(x) eine Beobachtungsfunktion, wobei die Funktionen f, g und h- anders als bei einem klassischen Kalman-Filter- keine linearen Funktionen sein müssen.

Im Fall der Blutpumpe 300 mit Back-EMF-Messung, axialer Lagerung und Rotationsregelung enthält der Messgrößenvektor y die gemessenen Induktionsspannungen, der Stellgrößenvektor u enthält das Drehmoment $M_\theta$ und die Lagerkraft Fz:

$$y = \begin{bmatrix} V_a \\ V_b \end{bmatrix} \qquad \text{(Gleichung 6a),}$$

$$u = \begin{bmatrix} M_\theta \\ F_Z \end{bmatrix} \qquad \text{(Gleichung 6b).}$$

Für die gemessenen Induktionsspannungen können Messgleichungen wie folgt hergeleitet werden. Das Magnetfeld B($\theta$,z) des Rotors 320 in Axialrichtung ist auf einem Kreis mit Radius r mit Abstand z von der Magnetoberfläche näherungsweise gegeben durch

$$B(\theta, z) = B_0 \sin(\theta)\, e^{-zk} = B_0 \sin\left(n_{ppz}\theta_{mech}\right) e^{-zk} \qquad \text{(Gleichung 7),}$$

wobei $B_0$ eine magnetische Feldstärke bei z = 0, $\theta$ ein elektrischer Rotationswinkel, $\theta_{mech}$ ein mechanischer Rotationswinkel, $n_{ppz}$ eine Polzahl des BLDC-Motors und k eine Wellenzahl ist. Der Rotor 320 erzeugt also ein entlang der Lagerwirkrichtung nichtlineares, insbesondere näherungsweise exponentiell abklingendes, Permanentmagnetfeld. Die Näherung gilt für ein sinusförmiges Magnetfeld, welches sich insbesondere bei eisenlosen Motoren oder Motoren mit großem Luftspalt einstellt. Blutpumpen sind häufig mit Motoren mit relativ großem Luftspalt ausgestattet, da im Luftspalt Fluid gefördert oder zumindest überbrückt werden muss. Die dargelegte Methode ist auch anwendbar auf nicht sinusförmige Magnetfelder, insbesondere trapezförmige Magnetfeldverläufe oder Magnetverläufe bestehend aus einer Linearkombination von mehreren harmonischen sinusförmigen Komponenten.

Mit der Definition $B_{mag} = B_0 e^{-zk}$ kann die zweiphasige Back-EMF dargestellt werden als

$$B_a = B_{mag} \sin(\theta) \qquad \text{(Gleichung 8a),}$$

$$B_b = B_{mag} \cos(\theta) \qquad \text{(Gleichung 8b).}$$

Mithilfe des Induktionsgesetzes (Gleichung 1) ergeben sich dann die folgenden Messgleichungen:

$$\frac{V_a}{NA} = -\left(\frac{dB_{mag}}{dt}\sin(\theta) + B_{mag}\frac{d\theta}{dt}\cos(\theta)\right) \qquad \text{(Gleichung 9a),}$$

$$\frac{V_b}{NA} = -\left(\frac{dB_{mag}}{dt}\cos(\theta) - B_{mag}\frac{d\theta}{dt}\sin(\theta)\right) \qquad \text{(Gleichung 9b).}$$

Zurückkehrend zum Modell des EKF-basierten Beobachters, können dem Zustandsvektor s entsprechend der in Gleichungen 7a und 7b enthaltenen Abhängigkeiten folgende Einträge zugewiesen werden:

$$x = \begin{bmatrix} B_{mag} \\ \dfrac{dB_{mag}}{dt} \\ \theta \\ \dfrac{d\theta}{dt} \end{bmatrix} \qquad \text{(Gleichung 10)}.$$

Verschiedene Einflüsse wie Fertigungstoleranzen, Temperaturänderungen oder Veränderungen der Viskosität des Fluids sorgen dafür, dass die Systemdynamik der Regelstrecke eine Unsicherheit ausweist. Die Systemdynamik wird jedoch explizit in den Gleichungen benötigt für die Übertragung der Stellgrößen auf Winkel und Position des Rotors 320. Wenn das mathematische Modell von der Wirklichkeit abweicht, treten dadurch u. U. große Schätzfehler auf. Dieser Punkt spricht demnach dagegen, die Stellgrößen im EKF zu berücksichtigen. Auch dagegen spricht, dass es nicht messbare Störungen gibt, die auf das System wirken, und deren Auswirkungen trotzdem vom EKF geschätzt werden müssen.

Allerdings kann eine Unterscheidung getroffen werden zwischen der Stellgröße Drehmoment und der Stellgröße Lagerkraft. Die Dynamik von Lagerkraft auf Lagerposition ist deutlich komplexer als die Dynamik von Drehmoment auf Drehwinkel, da der geschlossene Regelkreis berücksichtigt werden muss. Während zudem die Lagerkraftvorgabe typischerweise bei Null liegt (s. Diskussion der Nullkraftregelung weiter oben) und der Regler in diesem Fall nur Störungen auszugleichen hat, ist die Vorgabe des Drehmoments gerade bei großen Drehzahlen sehr groß und soll sich für schnelle Drehzahländerungen auch schnell ändern. Aus diesen Gründen bietet sich auch das folgende System für das EKF an, bei dem nur das Drehmoment als Stellgröße berücksichtigt wird:

$$x = \begin{bmatrix} B_{mag} \\ \dfrac{dB_{mag}}{dt} \\ \theta \\ \dfrac{d\theta}{dt} \\ K_M \end{bmatrix} \qquad \text{(Gleichung 10')},$$

$$u = M_\theta \qquad \text{(Gleichung 6b')}.$$

Herbei ist vorgesehen, dass der Zustandsvektor einen Drehmomentverstärkungsfaktor $K_M$ umfasst, der einem Verhältnis der Rotationsgeschwindigkeit und des Drehmoments entspricht.

Für die Auslegung eines Beobachters, insbesondere aber nicht ausschließlich im Fall des beispielhaft beschriebenen EKF-basierten Beobachters, sind vorteilhaft die folgenden Überlegungen zur Kopplung zwischen der Schätzung der Drehzahl und der Schätzung der Lagergeschwindigkeit zu berücksichtigen.

Die Messgleichungen (Gleichungen 7a, 7b) lassen sich auch in der folgenden Form schreiben:

$$V_a = -NA\,B_0 e^{-kz}\left(-k\,\dot{z}\sin(\theta) + \dot{\theta}\cos(\theta)\right) \qquad \text{(Gleichung 9a')},$$

$$V_b = -NA\,B_0 e^{-kz}\left(-k\,\dot{z}\cos(\theta) - \dot{\theta}\sin(\theta)\right) \qquad \text{(Gleichung 9b')}.$$

Hierbei wurden die zeitlichen Ableitungen $\dfrac{d\theta}{dt}$ bzw. $\dfrac{dz}{dt}$ als $\dot{\theta}$ bzw. $\dot{z}$ dargestellt. Es werden nun die folgenden Annahmen gemacht: der Beobachter sei konvergiert, womit z und $\theta$ bekannt sind; die geschätzten Werte der Rotationsgeschwindigkeit und der Lagergeschwindigkeit seien identisch den geschätzten Werten des vorherigen Zeitschritts. Es gelten:

$$\theta_e = \theta, \ \dot{\theta}_e = \dot{\theta} + \dot{\theta}_c, z_e = z, \ \dot{z}_e = \dot{z} + \dot{z}_c \qquad \text{(Gleichungen 11)},$$

wobei der Index e die geschätzten Werte, der Index c die Abweichung vom jeweiligen wahren Wert bezeichnet. Werden aus den geschätzten Werten nach Gleichungen 9 durch Einsetzen in die Gleichungen 7a', 7b' geschätzte Werte $V_{a,e}$, $V_{b,e}$ für die Induktionsspannungen berechnet und die Differenz mit den gemessenen Werten gebildet, so ergeben sich die Abweichungen

$$\Delta a = V_{a,e} - V_a = NA\,B_0 e^{-kz}\big(k\,\dot{z}_c\,\sin(\theta) - \dot{\theta}_c\,\cos(\theta)\big) \qquad \text{(Gleichung 12a)},$$

$$\Delta b = V_{b,e} - V_b = NA\,B_0 e^{-kz}\big(k\,\dot{z}_c\,\cos(\theta) + \dot{\theta}_c\,\sin(\theta)\big) \qquad \text{(Gleichung 12b)},$$

so kann die vorzunehmende Korrektur der Geschwindigkeiten (gleich dem Wert der Abweichung) wie folgt dargestellt werden:

$$\dot{z}_c = \frac{\Delta a\,\sin(\theta) + \Delta b\,\cos(\theta)}{kNAB_0 e^{-kz}} \qquad \text{(Gleichung 13a)},$$

$$\dot{\theta}_c = \frac{\Delta b\,\sin(\theta) - \Delta a\,\cos(\theta)}{NAB_0 e^{-kz}} \qquad \text{(Gleichung 13b)}.$$

Bei hinreichend kleiner Abtastzeit sind die obigen Annahmen und resultierenden Näherungen gerechtfertigt. Es verbleibt dennoch ein signifikanter Schätzfehler der Lagerposition z, wenn sich die Rotationsgeschwindigkeit stark ändert, da dann die Schätzung auf einer nicht mehr zutreffenden Rotationsgeschwindigkeit des vorherigen Zeitschritts basiert, was zu einer fehlerbehafteten Schätzung des Winkels führt.

Die Auslegung muss daher im Hinblick darauf erfolgen, welche maximalen Änderungen der Lagergeschwindigkeit und welche maximalen Änderungen der Rotationsgeschwindigkeit zwischen zwei Zeitschritten zu erwarten sind und wie sich die entsprechenden Schätzfehler der Rotationsgeschwindigkeit und der Lagergeschwindigkeit auf die weiteren zu schätzenden Größen auswirken.

Bei Weglassen der Annahme, dass Winkel und Lagerposition bekannt sind (d. h. es wird davon ausgegangen, dass auch Winkel und Lagerposition mit Schätzfehlern behaftet sind), kann eine zeitdiskrete Entsprechung der Gleichungen 9 wie folgt angegeben werden:

$$\theta_e(k) = \theta_e(k-1) + \dot{\theta}_e(k-1)\,T, \qquad \text{(Gleichung 14a)},$$

$$\dot{\theta}_e(k) = \dot{\theta}_e(k-1) = \dot{\theta} + \dot{\theta}_c, \qquad \text{(Gleichung 14b)},$$

$$z_e(k) = z_e(k-1) + \dot{z}_e(k-1)\,T, \qquad \text{(Gleichung 14c)},$$

$$\dot{z}_e(k) = \dot{z}_e(k-1) = \dot{z} + \dot{z}_c \qquad \text{(Gleichung 14d)}.$$

Daraus kann nun wiederum die geschätzte vorzunehmende Korrektur der Lagergeschwindigkeit gewonnen werden:

$$\dot{z}_{c,e} = \frac{\Delta a_e\,\sin(\theta_e) + \Delta b_e\,\cos(\theta_e)}{kNAB_0 e^{-kz_e}} \qquad \text{(Gleichung 15a)},$$

wobei für $\Delta a_e$ gilt:

$$\Delta a_e = -NA\, B_0 e^{-kz_e}\left(-\mathrm{k}\,\dot{z}_e\,\sin(\theta_e) + \dot{\theta}_e\,\cos(\theta_e)\right) +$$
$$NA\, B_0 e^{-kz}\left(-\mathrm{k}\dot{z}\,\sin(\theta) + \dot{\theta}\,\cos(\theta)\right) \qquad \text{(Gleichung 15b)}$$

($\Delta b_e$ analog).

Ist die Lagergeschwindigkeit konstant und bekannt, gilt also $z = z_e = 0$ und $\dot{z} = \dot{z}_e = 0$, so folgt:

$$\dot{z}_{c,e} = \frac{\dot{\theta}}{kNAB_0}\sin(\theta_e - \theta) \qquad \text{(Gleichung 16)}.$$

Ein Schätzfehler des Rotorwinkels führt demnach zu einem Fehler der Lagergeschwindigkeit. Eine schnelle Änderung der Rotationsgeschwindigkeit führt bei gleicher Dynamik des Beobachters zu größeren Schätzfehlern der Lagergeschwindigkeit. Da große Schätzfehler der Lagerposition irgendwann zwangsläufig zum Anschlagen des Rotors 320 an einer Wand des Gehäuses 301 führen muss die Änderungsgeschwindigkeit der Rotorgeschwindigkeit begrenzt werden.

Im Fall des EKF muss eine Q-Matrix, die das Systemrauschen beschreibt, so aufgebaut sein, dass insbesondere die unsicheren Zustandsparameter Lagergeschwindigkeit und Drehzahl schnell korrigiert werden. Für die verbleibenden Zustandsparameter reicht eine langsamere Korrektur aus.

In einem dritten Beispiel der Pumpenregelung ist die Steuereinheit 200 dazu eingerichtet, die Zustandsparameter statt mittels des EKF unter Verwendung eines manuell ausgelegten Beobachters zu schätzen. Dieser soll dabei im Wesentlichen das Verhalten des EKF-basierten Beobachters approximieren, was insbesondere den erforderlichen Rechenaufwand verringern kann.

Bei der manuellen Auslegung soll eine Beobachterverstärkung K bestimmt werden, die einen Schätzfehler $y_e$ des Messgrößenvektors y in jedem Zeitschritt mit einer Zustandskorrektur $\Delta x$ des Zustandsvektors x verknüpft:

$$\Delta x = K y_e \qquad \text{(Gleichung 17)}.$$

Die mittels des Beobachters zu lösende Schätzaufgabe kann in drei Teilaufgaben unterteilt werden: 1. Anpassung von $\dfrac{dB_{mag}}{dt}$ und $\dfrac{d\theta}{dt}$; 2. Anpassung von $B_{mag}$ und $\theta$, wobei diese Anpassung langsamer erfolgt als die Anpassung von $\dfrac{dB_{mag}}{dt}$ und $\dfrac{d\theta}{dt}$; 3. Schätzung von $K_M$.

Für jeden Zustandsparameter $x_i$ kann dann eine maximale Verstärkung $k_i$ angegeben werden, mit der die Anpassung erfolgt:

$$\Delta x_i = \sum_{j=1}^{2} k_i\, e_{ij} y_{e,j}, \quad 0 < e_{ij} \leq 1 \qquad \text{(Gleichung 18)}.$$

Für die Bestimmung der in Gleichung 16 enthaltenen Skalierungswerte $e_{ij}$ werden zwei alternative Verfahren vorgeschlagen.

In dem ersten Verfahren werden die $e_{ij}$ vorab aus den Messgrößen $y_i$ bestimmt. Hierzu wird eine komplexe Zahl Y und eine normierte komplexe Zahl F mittels der gemessenen Induktionsspannungen definiert:

$$Y = V_a + i\, V_b \qquad \text{(Gleichung 19a)},$$

$$F = \frac{Y}{|Y|} \qquad \text{(Gleichung 19b)}.$$

Basierend auf einer empirisch gewählten Vorgabe von komplexen Zahlen fij können die $e_{ij}$ dann berechnet werden als

$$e_{ij} = real(f_{ij}\, F) \qquad\qquad \text{(Gleichung 20).}$$

In dem zweiten Verfahren für die Bestimmung der $e_{ij}$ werden die $e_{ij}$ getrennt nach den obigen Teilaufgaben optimiert. Beispielsweise kann für die Optimierung bei der ersten Teilaufgabe eine Basis, zum Beispiel die Basis

$$\begin{bmatrix} e_2 \\ e_4 \end{bmatrix} = \begin{bmatrix} \sin(\frac{2\pi k}{N}) \\ \cos(\frac{2\pi k}{N}) \end{bmatrix}, \quad k \in \{1,2,\dots,N\} \qquad \text{(Gleichung 21)}$$

vorgegeben und dann in jedem Schritt ein k gewählt werden, das den Messfehler minimiert. Durch geeignete Wahl der Anzahl N der Basisvektoren kann ein gewünschter Kompromiss zwischen Rechenaufwand und Schätzgenauigkeit eingestellt werden.

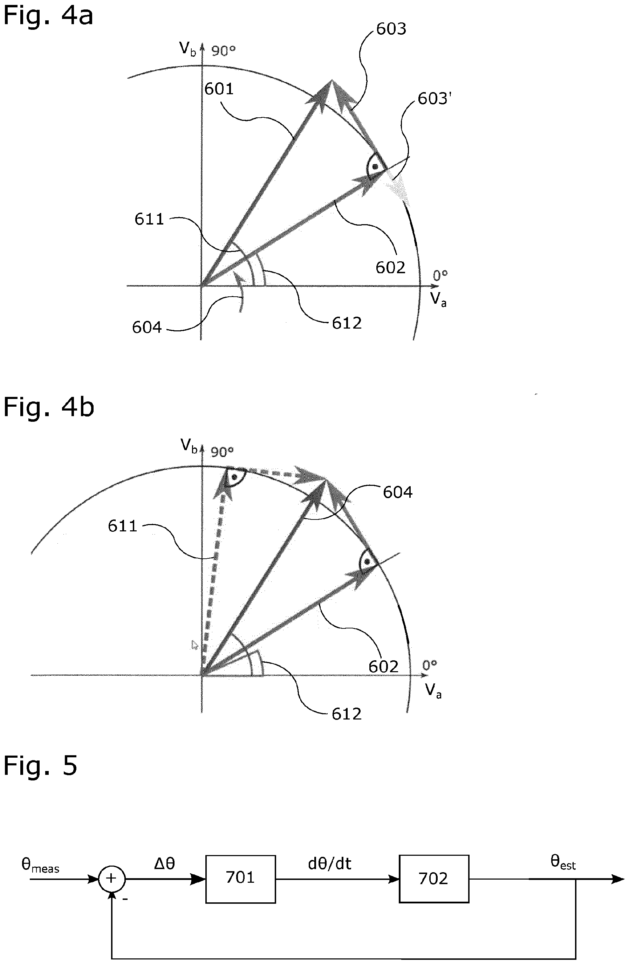

In einem vierten Beispiel der Pumpenregelung, das in Fig. 4a, Fig. 4b und Fig. 5 illustriert wird, ist die Steuereinheit 200 dazu eingerichtet, die Zustandsparameter auf besonders einfache Weise unter Verwendung einer vektoriellen Zerlegung der gemessenen Back-EMF in eine Rotationskomponente und eine Lagerpositionskomponente zu schätzen.

Fig. 4a zeigt ein Zeigerdiagramm für eine induzierte Back-EMF, dargestellt als Back-EMF-Vektor 601 in einer zweiphasigen Darstellung, wobei diese Darstellung insbesondere mittels d/q-Transformation aus einer dreiphasigen messbaren Back-EMF erzeugt werden kann. Der Back-EMF-Vektor kann in eine Rotationskomponente 602 und eine dazu senkrechte Lagerpositionskomponente 603 zerlegt, d. h. als vektorielle Summe dieser beiden Komponenten dargestellt, werden.

Der Betrag der Rotationskomponente 602 ist $NA\, B_{mag}\, \frac{d\theta}{dt}$, also insbesondere proportional zur Drehzahl des Rotors 320. Der Phasenwinkel 604 der Rotationskomponente 602 entspricht dem elektrischen Rotationswinkel. Die Rotationskomponente 602 rotiert im Zeigerdiagramm entsprechend der elektrischen Rotordrehzahl (Drehrichtung 605).

Die Lagerpositionskomponente 603 steht senkrecht auf der Rotationskomponente 602 und hat den Betrag $NA\, \frac{dB_{mag}}{dt}$ und ist damit proportional zur translatorischen Rotorbewegung. Die an Anschlüssen der Spulen der Motorspulenanordnung 343 messbare Induktionsspannung entspricht dem Betrag des Back-EMF-Vektors 601. Da sich dieser als vektorielle Summe der Rotationskomponente 602 und der Lagerpositionskomponente 603 darstellen lässt, kann der folgende Ausdruck abgelesen werden:

$$\sqrt{\frac{1}{(NA)^2}(V_a^2 + V_b^2) - B_{mag}^2 \left(\frac{d\theta}{dt}\right)^2} = \left| \frac{dB_{mag}}{dt} \right| \quad \text{(Gleichung 22).}$$

Aus dieser Beziehung ergibt sich nur der Betrag, nicht aber das Vorzeichen der Magnetfeldänderung. Es sind also beide in Fig. 4b dargestellten Konstellationen möglich (gestrichelte bzw. durchgezogene Pfeile). Für die Bestimmung des Vorzeichens ist zusätzlich eine Schätzung des elektrischen Rotationswinkels $\theta$ erforderlich.

Hierzu kann einerseits die anmeldungsgemäß entkoppelte Dynamik von Lagerpositionsregelung und Rotationsregelung ausgenutzt werden, andererseits die Tatsache, dass $\frac{dB_{mag}}{dt}$ im zeitlichen Mittel Null ist, da der Rotor 320 sich nur innerhalb eines begrenzten Auslenkungsbereichs entlang der Lagerwirkrichtung aufhalten kann. Eine Schätzung der Rotationsgeschwindigkeit und des Rotationswinkels ist daher mittels eines einfachen PID-Regelkreises, umfassend einen PID-Regler 701 und einen Integrator 702, möglich, der in Fig. 5 dargestellt ist.

Das Vorzeichen von $\frac{dB_{mag}}{dt}$ kann dann entsprechend der Darstellung in Fig. 4b bestimmt werden, indem ermittelt wird, welche der beiden möglichen Konstellationen näher an dem geschätzten Rotationswinkel 606 ($\theta_{est}$) liegt. Da ferner die Rotationskomponente 602 senkrecht zur Lagerpositionskomponente 603 steht, kann die Magnetfeldänderung schließlich berechnet werden als

$$\frac{dB_{mag}}{dt} = -\frac{1}{NA}\left[\sin(\theta_{est})\,V_a + \cos(\theta_{est})V_b\right] \qquad \text{(Gleichung 23)}.$$

Die Steuereinheit 200 kann - unabhängig davon, wie die Pumpenregelung implementiert ist - ferner dazu eingerichtet sein,

eine Mehrzahl von Messpunkten, entsprechend wenigstens eines Anteils des Messsignals zu einer Mehrzahl von Zeitpunkten bei konstanter Rotationsgeschwindigkeit und konstanter Lagerposition des Rotors 320 zu erfassen, eine periodische Korrekturfunktion, insbesondere eine Sinusfunktion oder mehrere überlagerte Sinusfunktionen, an wenigstens einen Anteil des Messsignals mittels Optimierung anzupassen.

Vorzugsweise wird an jede Induktionsspannung des mehrdimensionalen Back-EMF-Messsignals eine entsprechende periodische Korrekturfunktion angepasst. Die erforderliche konstante Axialposition kann dabei beispielsweise unter Verwendung eines zusätzlichen Sensors, etwa eines Hall-Sensors, eingeregelt werden. Im hier beschriebenen Beispiel wird an jede Induktionsspannung eine Sinusfunktion mit gemeinsamer Amplitude A, Kreisfrequenz $\omega$ und Phase $\varphi_0$ angepasst, wobei die Sinusfunktionen sich um einen festen Phasenversatz entsprechend einer Anzahl von Spulenpaare der Motorspulenanordnung 343 unterscheiden. Bei drei Spulenpaaren und entsprechenden Induktionsspannungen $V_A$, $V_B$, Vc beträgt der Phasenversatz 120 °. In diesem Fall lautet ein zu minimierendes Gütekriterium (figure of merit) für die Optimierung:

$$I = \sum_{k=0}^{N}(A\sin(\omega kT + \varphi_0) - V_A(k))^2 + \left(A\sin\left(\omega kT + \varphi_0 + \frac{2}{3}\pi\right) - V_B(k)\right)^2 + \left(A\sin\left(\omega kT + \varphi_0 + \frac{4}{3}\pi\right) - V_C(k)\right)^2 \qquad \text{(Gleichung 24)}.$$

Die Steuereinheit 200 ist in diesem Beispiel dazu eingerichtet, auf Grundlage der so angepassten Korrekturfunktion eine von dem Rotationswinkel des Rotors abhängige, insbesondere periodisch mit einer Periode der Rotation des Rotors auftretende, Störung des Messsignals auszugleichen, so dass somit deren Einfluss auf das Steuersignal verringert wird.

Liste der Bezugszeichen

| | |
|---|---|
| 100 | Pumpensystem, |
| 200 | Steuereinheit, |
| 300 | Blutpumpe, |
| 301 | Gehäuse, |
| 302 | Einlass, |
| 303 | Auslass, |
| 304 | Volute, |
| 305 | Driveline, |
| 320 | Rotor, |
| 321 | Beschaufelung, |
| 322 | einlassseitiges Rotormagnetlager, |
| 323 | auslassseitiges Rotormagnetlager, |
| 324 | Motormagnetanordnung, |
| 340 | Stator, |
| 341 | einlassseitiges Statormagnetlager, |
| 342 | auslassseitiges Statormagnetlager, |
| 343 | Motorspulenanordnung, |
| 344 | Steuerspule, |
| 401,402,403,404 | Messsignalkurve, |
| 501 | Regler, |
| 502 | Regelstrecke, |
| 503 | Beobachter, |

| 601 | Back-EMF-Vektor, |
| 602 | Rotationskomponente, |
| 603 | Lagerpositionskomponente, |
| 604 | Phasenwinkel, |
| 605 | Drehrichtung, |
| 606 | geschätzter Rotationswinkel, |
| 701 | PID-Regler, |
| 702 | Integrator, |
| X | Rotationsachse. |

**Patentansprüche**

1. Steuereinheit (200) für eine Blutpumpe (300),

   wobei die Blutpumpe (300) einen in einem Gehäuse (301) magnetisch gelagerten und zum Fördern eines Fluids um eine Rotationsachse (X) rotierbaren Rotor (320) und einen Stator (340) umfasst, wobei der Stator (340) eingerichtet ist zum Erzeugen eines variierbaren Statormagnetfelds zum Ausüben einer variierbaren Lagerkraft entlang einer Lagerwirkrichtung und eines Drehmoments bezüglich der Rotationsachse (X) auf den Rotor (320); wobei die Blutpumpe (300) eingerichtet ist zum Bereitstellen eines Messsignals, umfassend einen von einer Lagerposition und/oder Lagergeschwindigkeit einer Bewegung des Rotors (320) entlang der Lagerwirkrichtung abhängigen Anteil und einen von einem Rotationswinkel und/oder einer Rotationsgeschwindigkeit einer Rotation des Rotors (320) um die Rotationsachse (X) abhängigen Anteil; wobei die Steuereinheit (200) dazu eingerichtet ist,

   basierend auf dem Messsignal ein Steuersignal zum Variieren des Statormagnetfelds derart zu bestimmen, dass der Rotor (320) mittels der Lagerkraft berührungsfrei entlang der Lagerwirkrichtung in dem Gehäuse (301) gelagert und mittels des Drehmoments eine Rotation des Rotors (320) um die Rotationsachse (X) erzeugt, insbesondere gesteuert und/oder geregelt, wird, wobei eine Vorgabe für eine Änderungsrate der Rotationsgeschwindigkeit der Rotation des Rotors (320) um die Rotationsachse (X) durch eine vorgegebene Maximaländerungsrate nach oben begrenzt ist, um einen Einfluss einer Änderung der Rotationsgeschwindigkeit auf das bestimmte Steuersignal zu begrenzen, und den Stator (340) gemäß dem bestimmten Steuersignal anzusteuern.

2. Steuereinheit (200) nach Anspruch 1, wobei die vorgegebene Maximaländerungsrate so gewählt ist, dass der Einfluss der Änderung der Rotationsgeschwindigkeit durch das Ansteuern des Stators (340) gemäß dem bestimmten Steuersignal auf das Steuersignal, insbesondere auf eine basierend auf dem Messsignal vorgenommene und in das Steuersignal eingehende Bestimmung der Lagerposition und/oder Lagergeschwindigkeit, begrenzt wird gegenüber einem Ansteuern des Stators (340) gemäß einem Steuersignal, das ohne Begrenzen der Änderungsrate der Rotationsgeschwindigkeit auf die vorgegebene Maximaländerungsrate bestimmbar ist.

3. Steuereinheit (200) nach einem der vorhergehenden Ansprüche, wobei die vorgegebene Maximaländerungsrate proportional zu einem Dynamik-Parameter, der Bewegung des Rotors (320) entlang der Lagerwirkrichtung ist, wobei der Dynamik-Parameter insbesondere ein Parameter einer Steuerung oder Regelung der Bewegung des Rotors (320) entlang der Lagerwirkrichtung und/oder eine basierend auf dem Messsignal bestimmte Größe ist.

4. Steuereinheit (200) nach Anspruch 3, wobei der Dynamik-Parameter eine Lagerbeschleunigung und/oder Auslenkungsfrequenz und/oder Auslenkungsfrequenz-Änderungsrate der Bewegung des Rotors (320) entlang der Lagerwirkrichtung und/oder eine Durchtrittsfrequenz und/oder Resonanzfrequenz einer Regelung der Bewegung des Rotors entlang der Lagerwirkrichtung ist.

5. Steuereinheit (200) nach einem der vorhergehenden Ansprüche, wobei die vorgegebene Maximaländerungsrate durch eine Zeitkonstante, insbesondere eine Verzugszeit und/oder Sprungantwortzeit und/oder Anstiegszeit, einer Regelung der Rotation des Rotors (320) gegeben ist.

6. Steuereinheit (200) nach einem der vorhergehenden Ansprüche, wobei die vorgegebene Maximaländerungsrate höchstens die Hälfte, vorzugsweise höchstens ein Fünftel, insbesondere höchstens ein Zehntel einer charakteristischen Änderungsrate der Bewegung des Rotors (320) entlang der Lagerwirkrichtung beträgt und/oder wobei eine charakteristische Zeitkonstante, durch die die vorgegebene Maximaländerungsrate gegeben ist, mindestens das Doppelte, vorzugsweise das Fünffache, insbesondere mindestens das Zehnfache einer charakteristischen Zeitkonstante der Bewegung des Rotors (320) entlang der Lagerwirkrichtung beträgt.

7. Steuereinheit (200) nach einem der vorhergehenden Ansprüche, wobei das Messsignal einer aufgrund einer durch die Rotation des Rotors (320) und/oder die Bewegung des Rotors (320) entlang der Lagerwirkrichtung verursachten magnetischen Feldänderung im Stator (340) induzierten elektromotorischen Gegenkraft (Back-EMF) entspricht.

8. Steuereinheit (200) nach einem der vorhergehenden Ansprüche, dazu eingerichtet,

   basierend auf dem Messsignal einen oder mehrere Zustandsparameter eines Modells der Bewegung des Rotors (320), insbesondere der Rotation des Rotors (320) um die Rotationsachse und/oder der Bewegung des Rotors (320) entlang der Lagerwirkrichtung, zu schätzen, wobei der bzw. die Zustandsparameter insbesondere den Rotationswinkel und/oder die Rotationsgeschwindigkeit und/oder die Änderungsrate der Rotationsgeschwindigkeit und/oder eine Rotationsfrequenz und/oder eine Rotationsfrequenz-Änderungsrate der Rotation des Rotors (320) und/oder die Lagerposition und/oder die Lagergeschwindigkeit und/oder eine Lagerbeschleunigung und/oder eine Auslenkungsfrequenz und/oder eine Auslenkungsfrequenz-Änderungsrate der Bewegung des Rotors (320) entlang der Lagerwirkrichtung und/oder die Lagerkraft und/oder das Drehmoment und/oder eine Feldstärke eines innerhalb des Stators (340) durch den Rotor (320) erzeugten Magnetfeldes und/oder eine zeitliche Änderung dieser Feldstärke umfassen und
   das Steuersignal basierend auf einem oder mehreren des bzw. der geschätzten Zustandsparameter zu bestimmen.

9. Steuereinheit (200) nach Anspruch 8, wobei der bzw. die Zustandsparameter einen Drehmomentverstärkungsfaktor umfassen, der einem Verhältnis der Rotationsgeschwindigkeit und des Drehmoments entspricht.

10. Steuereinheit (200) nach Anspruch 8 oder 9, dazu eingerichtet, den bzw. die Zustandsparameter unter Verwendung eines Extended Kalman Filters zu schätzen.

11. Steuereinheit (200) nach einem der Ansprüche 8 bis 10, dazu eingerichtet, den bzw. die Zustandsparameter unter Verwendung eines manuell ausgelegten Beobachters und/oder unter Verwendung einer vektoriellen Zerlegung einer gemessenen Back-EMF in eine Rotationskomponente (602) und eine Lagerpositionskomponente (603) zu schätzen.

12. Steuereinheit (200) nach einem der Ansprüche 8 bis 11, wobei die Steuereinheit (200) dazu eingerichtet ist, dass Steuersignal so zu bestimmen, dass der Rotor (320) in eine Ziel-Lagerposition eingeregelt wird, an der auf den Rotor (320) wirkende äußere Kräfte entlang der Lagerwirkrichtung sich zu einer vorgegebenen Kraft, insbesondere einer Nullkraft, addieren und/oder an der eine zum Erzeugen des variierbaren Statormagnetfelds aufgewandte Leistung minimal ist.

13. Steuereinheit (200) nach einem der Ansprüche 8 bis 12, wobei eine Mehrzahl von Messpunkten, entsprechend dem Messsignal zu einer Mehrzahl von Zeitpunkten, erfasst wird und die Steuereinheit (200) dazu eingerichtet ist, das Steuersignal basierend auf der Mehrzahl von Messpunkten zu bestimmen.

14. Steuereinheit (200) nach einem der Ansprüche 8 bis 13, ferner dazu eingerichtet,

   eine Mehrzahl von Messpunkten, entsprechend wenigstens eines Anteils des Messsignals zu einer Mehrzahl von Zeitpunkten bei konstanter Rotationsgeschwindigkeit und bekannter, insbesondere konstanter, Lagerposition des Rotors (320) zu erfassen,
   eine periodische Korrekturfunktion, insbesondere eine Sinusfunktion oder mehrere überlagerte Sinusfunktionen, an wenigstens einen Anteil des Messsignals mittels Optimierung anzupassen.

15. Pumpensystem, umfassend

   eine Blutpumpe (300),

      wobei die Blutpumpe (300) einen in einem Gehäuse (301) magnetisch gelagerten und zum Fördern eines Fluids um eine Rotationsachse (X) rotierbaren Rotor (320) und einen Stator (340) umfasst, wobei der Stator (340) eingerichtet ist zum Erzeugen eines variierbaren Statormagnetfelds zum Ausüben einer variierbaren Lagerkraft entlang einer Lagerwirkrichtung und eines Drehmoments bezüglich der Rotationsachse (X) auf den Rotor (320);

wobei die Blutpumpe (300) eingerichtet ist zum Bereitstellen eines Messsignals, umfassend einen von einer Lagerposition und/oder Geschwindigkeit einer Bewegung des Rotors (320) entlang der Lagerwirkrichtung abhängigen Anteil und einen von einem Rotationswinkel und/oder einer Rotationsgeschwindigkeit einer Rotation des Rotors (320) um die Rotationsachse (X) abhängigen Anteil; und

eine Steuereinheit (200) gemäß einem der Ansprüche 8 bis 14, eingerichtet zum Ansteuern der Blutpumpe (300).

16. Pumpensystem nach Anspruch 15, wobei der Rotor (320) und der Stator (340) einen Axialflussmotor oder Radialflussmotor bilden und die Lagerwirkrichtung im Wesentlichen parallel zu der Rotationsachse (X) verläuft.

17. Pumpensystem nach Anspruch 15 oder 16, wobei der Rotor (320) und der Stator (340) einen Radialflussmotor bilden und die Lagerwirkrichtung in einem von Null verschiedenen Winkel, insbesondere im Wesentlichen senkrecht, zu der Rotationsachse (X) des Rotors (320) stehenden Radialrichtung steht, wobei der Rotor (320) vorzugsweise neben der vorgenannten Lagerwirkrichtung auch entlang einer zweiten Lagerwirkrichtung aktiv magnetisch in dem Gehäuse (301) gelagert ist.

18. Pumpensystem nach einem der Ansprüche 15 bis 17, wobei der Rotor (320) ein entlang Lagerwirkrichtung und/oder der Rotationsachse (X) nichtlineares, insbesondere wenigstens näherungsweise exponentiell abklingendes, Permanentmagnetfeld erzeugt.

19. Verfahren zum Ansteuern einer Blutpumpe (300),

wobei die Blutpumpe (300) einen in einem Gehäuse (301) magnetisch gelagerten und zum Fördern eines Fluids um eine Rotationsachse (X) rotierbaren Rotor (320) und einen Stator (340) umfasst, wobei der Stator (340) eingerichtet ist zum Erzeugen eines variierbaren Statormagnetfelds zum Ausüben einer variierbaren Lagerkraft entlang einer Lagerwirkrichtung entlang der Lagerwirkrichtung;

wobei die Blutpumpe (300) eingerichtet ist zum Bereitstellen eines Messsignals, umfassend eine von einer Lagerposition und/oder Geschwindigkeit einer Bewegung des Rotors (320) entlang der Lagerwirkrichtung abhängigen Anteil und einen von einem Rotationswinkel und/oder einer Rotationsgeschwindigkeit einer Rotation des Rotors (320) um die Rotationsachse (X) abhängigen Anteil;

wobei das Verfahren umfasst:

Bestimmen, basierend auf dem Messsignal, eines Steuersignals zum Variieren des Statormagnetfelds derart, dass der Rotor (320) mittels der Lagerkraft berührungsfrei entlang der Lagerwirkrichtung in dem Gehäuse (301) gelagert und mittels des Drehmoments eine Rotation des Rotors (320) um die Rotationsachse (X) erzeugt, insbesondere gesteuert und/oder geregelt wird, wobei eine Vorgabe für eine Änderungsrate der Rotationsgeschwindigkeit der Rotation des Rotors (320) um die Rotationsachse (X) durch eine vorgegebene Maximaländerungsrate nach oben begrenzt ist, um einen Einfluss einer Änderung der Rotationsgeschwindigkeit auf das bestimmte Steuersignal zu begrenzen, und

Ansteuern des Stators (340) gemäß dem bestimmten Steuersignal.

## Fig. 1

## Fig. 2

## Fig. 3

## Fig. 4a

## Fig. 4b

## Fig. 5

Europäisches
Patentamt
European
Patent Office
Office européen
des brevets

**EUROPÄISCHER RECHERCHENBERICHT**

**Nummer der Anmeldung**

**EP 22 16 3254**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | EP 3 827 852 A1 (BERLIN HEART GMBH [DE]) 2. Juni 2021 (2021-06-02) | 1,2,5-8, 10-13,15 | INV. A61M60/122 |
| A | * Absätze [0028] – [0106]; Abbildungen 1, 11-19, 38-43 * | 3,4,9,14 | A61M60/216 A61M60/422 A61M60/538 |
| A | WO 2021/216960 A1 (VERITIUM RES LLC [US]) 28. Oktober 2021 (2021-10-28) * Absätze [0039] – [0042], [0095] – [0098]; Abbildungen 1, 28 * | 1 | A61M60/546 A61M60/822 |
| Y | US 2005/215843 A1 (MEDVEDEV ALEXANDER [US]) 29. September 2005 (2005-09-29) | 1,2,5-8, 10-13,15 | |
| A | * Absätze [0014] – [0029]; Abbildungen 1-5 * | 3,4,9,14 | |

RECHERCHIERTE SACHGEBIETE (IPC)

A61M

Der vorliegende Recherchenbericht wurde für alle Patentansprüche erstellt

| Recherchenort | Abschlußdatum der Recherche | Prüfer |
|---|---|---|
| Den Haag | 7. Dezember 2022 | Schlaug, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 22 16 3254

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-12-2022

| Im Recherchenbericht angeführtes Patentdokument | Datum der Veröffentlichung | Mitglied(er) der Patentfamilie | Datum der Veröffentlichung |
|---|---|---|---|
| EP 3827852 A1 | 02-06-2021 | CN 114746143 A | 12-07-2022 |
| | | DE 112020005772 T5 | 22-09-2022 |
| | | EP 3827852 A1 | 02-06-2021 |
| | | WO 2021105425 A2 | 03-06-2021 |
| WO 2021216960 A1 | 28-10-2021 | KEINE | |
| US 2005215843 A1 | 29-09-2005 | KEINE | |

EPO FORM P0461

Für nähere Einzelheiten zu diesem Anhang : siehe Amtsblatt des Europäischen Patentamts, Nr.12/82